# EUROPEAN PATENT APPLICATION

(11) **EP 4 534 088 A1**
(43) Date of publication of application: **09.04.2025**
(21) Application number: 23201510.7
(22) Date of filing: 04.10.2023
(51) Int. Cl.: A61K 31/519, A61K 31/4415, A61K 31/4745, A61K 31/513, A61K 31/555, A61K 39/00, A61K 45/06, A61K 9/00, A61P 35/00, A61P 35/04

(54) **ARFOLITIXORIN FOR USE IN VITAMIN B6-ENHANCED, 5-FLUOROURACIL-BASED CHEMOTHERAPY OF CARCINOMAS**

(71) Applicant: Isofol Medical AB, 413 46 Göteborg (SE)
(72) Inventor: LINDBERG, Per Lennart, 413 01 Göteborg (SE); Machover, David, 75005 Paris (FR)
(74) Representative: AWA Denmark A/S

(57) **Abstract**

The present invention relates to the treatment of carcinoma, such as colorectal cancer, in human populations employing a dosage regimen which includes arfolitixorin, i.e. [6R]-5,10-methylenetetrahydrofolate ([6R]-MTHF) and a vitamin B6 in connection with 5-fluorouracil (5-FU) based chemotherapy.

## Description

### BACKGROUND OF THE INVENTION

Colorectal cancer (CRC) is one of the leading causes of mortality and morbidity in the world. With approximately 1,849,518 new cases estimated and 880,792 deaths per year (Caputo 2019), it also represents the third most common cancer worldwide and the second cause of cancer-related mortality, after lung cancer. In terms of geographical distribution, CRC incidence and prevalence have risen in industrialized countries (Bray 2018). Colorectal cancer affects approximately 135.439 estimated new patients in the United States per year. Of these cases, 39.910 per year (30%) are due to rectal cancer (Recio-Boiles 2020). However, in recent years the incidence and mortality rates of CRC have grown higher in Eastern Europe, Latin America, and Asia than other countries.

In this setting, molecular alterations occurring in Kirsten rat sarcoma virus (*KRAS*)*,* neuroblastoma RAS viral oncogene homolog (*NRAS*)*,* and B-Raf proto-oncogene (*BRAF*) significantly worsen disease prognosis. The RAS oncogene family consists of three genes in humans, located in the short arm of chromosome 12, namely *KRAS, NRAS,* and Harvey rat sarcoma viral oncogene homolog (*HRAS*)*.* The RAS family is one of the most frequently mutated across all malignancies, including CRC. Indeed, around 40% of CRC harbors *KRAS* mutations plus an additional 4% *NRAS* mutations (and a negligible <1% prevalence of *HRAS* mutations), with >95% of them occurring in *KRAS* G12, G13, or Q61 codons. RAS and *BRAF* mutations confer more aggressive tumor biology, shorter OS in particular in microsatellite stable (MSS) mCRC, and are negative predictive factors for response to anti-epidermal growth factor receptor (EGFR) therapy (cetuximab or panitumumab). Accordingly, current clinical guidelines for RAS mutant (MT) MSS mCRC recommend chemotherapy (FOLFOX or FOLFIRI or FOLFOXIRI) with the addition of anti-vascular endothelial growth factor agents (bevacizumab in first line, bevacizumab or aflibercept in second line) as mainstream for early lines of treatment (Patelli 2021).

While new therapeutic strategies are emerging in other molecular subsets, like doublet or triplet combinations of anti-EGFR, anti-BRAF, and anti-MEK [mitogen-activated protein kinase 1 (MAPK1)] harnessing *BRAF^{V600E}* mutations, and checkpoint inhibitor immunotherapy in microsatellite unstable (MSI) mCRC, *KRAS*/*NRAS* mutations still represent the main clinical unmet need in this disease (Patelli 2021).

Conventional treatment for advanced CRC encompasses the combination of 5-fluorouracil (5-FU) and leucovorin with oxaliplatin or irinotecan. The medical treatment in CRC has made great strides with the advent of monoclonal antibodies such as bevacizumab and cetuximab. Despite the improvement in response rates with various modulation strategies such as monoclonal antibodies combined with chemotherapy, the five-year survival rate for mCRC is only slightly over 12 percent. One of the major obstacles for this observation is due to the appearance of drug resistance. Nearly half of mCRC patients are resistant to 5-FU-based chemotherapies including a unique drug resistance to 5-FU, which is closely related to the expression of thymidylate synthase (TS). Since TS is the primary target of 5-FU, patients with low TS expression display a better overall survival (OS) than patients with higher TS expression in tumor tissue. As TS is encoded by the TYMS gene, the level of TYMS gene expression offers significant prognostic value (Van der Jeught 2018).

CRC chemotherapy comprising fluoropyrimidines (5-FU and capecitabine) cause inhibition of *de novo* thymidine creation from uracil by the TYMS enzyme. Potential resistance mechanisms to fluoropyrimidines include TYMS gene amplification, loss of heterozygosity (LOH) and a negative feedback mechanism. The TYMS gene (GenelD 7298) is located on the short arm of chromosome 18 (18p11.32) and several polymorphisms of the TYMS gene have been connected to variable TYMS protein levels and therapeutic outcome in relation to 5-FU (Ntavatzikos 2017). Elevated expression of the TYMS gene is linked to acquired 5-FU resistance in CRC cells. (Varghese 2019).

In terms of treatment, RAS and in particular the *KRAS* mutation has been found to have an adverse impact on the prognosis for stage IV CRC patients treated with the FOLFOX regimen (Zocche 2015) or on stage II or III CRC patients treated with adjuvant FOLFOX (Lee 2014). In a study related to first-line treatment of mCRC, there was no evidence for patients whose tumors carried mutations in *KRAS* of a benefit associated with the addition of cetuximab to FOLFIRI in relation to PFS, overall survival, or best overall response (ORR). Patients in both treatment groups whose tumors carried mutations in *KRAS* appeared to have worse overall survival than those whose tumors were wild-type. The addition of cetuximab to FOLFIRI in patients with *KRAS* wild-type disease resulted on the other hand in significant improvements in overall survival (Van Cutsem 2011). *KRAS* mutations have further been found to confer resistance to epidermal growth factor receptor (EGFR) inhibitors, a class of tyrosine kinase inhibitors or monoclonal antibodies designed to slow or halt uncontrolled cell growth (Bai 2015).

CRC patients with BRAF-mutations do not benefit, either, from EGFR inhibitors. The current standard therapy in first-line treatment of BRAF-mutated CRC is folate/5-fluorouracil (5-FU) assisted oxaliplatin-based chemotherapy plus bevacizumab, increasingly including the more intensive "triplet" therapy FOLFOXIRI (i.e. leucovorin/5-FU/oxaliplatin/ irinotecan) plus bevacizumab, which however is only a valid option in patients with a good ECOG (Eastern Cooperative Oncology Group) performance status (Caputo 2019).

FOLFOXIRI, combined with bevacizumab or panitumumab have been shown to be superior in terms of early tumor shrinkage (ETS) and depth of response (DpR) compared to doublet combinations in patients with *RAS* wild-type metastatic colorectal cancer. The modified mFOLFOXIRI + cetuximab regimen was shown to be significantly superior to the mFOLFOXIRI + bevacizumab in terms of DpR as the primary endpoint in first-line treatment for RAS wild-type mCRC, whereas the two arms did not differ significantly with respect to ETS and ORR (Tsuji 2021).

Triplet chemotherapy has thus in many studies been found to be a good option for otherwise fit CRC patients with aggressive tumors, and has also been suggested for *KRAS-*mutated CRC cases (Glynne-Jones 2020).

However, triplet regimens such as FOLFOXIRI are in general associated with a highly increased level of adverse side effects. In a meta-analysis of patient data from 5 clinical studies it was thus found that - compared with patients receiving doublet therapy (FOLFOX or FOLFIRI) - patients in the triplet group (FOLFOXIRI plus bevacizumab) had higher rates of grade 3 or 4 neutropenia (45.8% vs 21.5°/, P < .001), febrile neutropenia (6.3% vs 3.7°/, P = .019), nausea (5.5% vs 3.0%, P = .016), mucositis (5.1% vs 2.9%, P = .024), and diarrhea (17.8% vs 8.4°/, P < .001). Death due to toxicity occurred in 2.3% vs 1.4% of patients (P = .277) (Cremolini 2020).

Experiments conducted in the human colon carcinoma HT29 and HCT116 cell lines, and in the murine leukemia L1210 cell line *in vitro* to investigate for interactions between 5-FU, leucovorin and the cofactor pyridoxal 5'-phosphate (PLP) on cell growth has shown that supplementation of cancer cells exposed to 5-FU with high concentrations of PLP and leucovorin potentiated the cytotoxic activity of 5-FU in the three cell lines (Machover 2018). In a later study (Machover 2021) five patients with advanced metastatic colorectal adenocarcinoma were treated according to the classical FOLFIRINOX triplet regimen (leucovorin, 5-FU, irinotecan, and oxaliplatin) supplemented with pyridoxine in high doses.

Antitumor activity was found to be high for several patients, but due to the small scale of the study, lack of controls and varied patient disease history it was not clear whether the co-administration of pyridoxine led to a significantly different result from what would have been expected from employing standard-of-care treatment on the same group of patients. Assessment of toxicity associated with the pyridoxine-modified protocols was however of the same magnitude as expected for the standard protocols.

Triplet regimens therefore remains recommended only for mCRC patients whose general condition is very good, estimated to around 40-50% of all mCRC patients (Cremolini 2019). For the majority of patients with metastatic CRC, there thus remains an unmet need for an improved folate/fluoropyrimidine-based treatment protocol of their disease, such as a more efficient and better tolerated triplet chemotherapeutic regimen.

### DEFINITIONS

As used herein, the term **Leucovorin^{®}** or **folinic acid** shall both mean 5-formyl tetrahydrofolic acid, i.e. the 5-formyl derivative of tetrahydrofolic acid. Folinic acid contains 2 asymmetric centers. **Leucovorin^{®}** (LV) is composed of a 1:1 mixture of the two dextrorotary and levorotary diastereomers (*d*-leucovorin (*d*-LV, (6R,2'S)-configuration) and *l*-leucovorin (*l*-LV, (6S,2'S)-configuration), respectively), and may also be referred to as **(d,*l*-LV).**

As used herein, the term **Levoleucovorin** shall refer to the commercially available product which contains only the natural and pharmacologically active *levo*-isomer *l*-LV (or LLV). In vitro, *l*-LV has been shown to be rapidly converted to the biologically available methyl-tetrahydrofolate form while the *dextro* form *d*-LV (DLV) is slowly excreted by the kidneys. Leucovorin and levoleucovorin have however been shown to be pharmacokinetically identical and may be used interchangeably with limited differences in efficacy (considering the inactive d-isomer in LV) or side effects (see Kovoor et al, Clin Colorectal Cancer 8 200-6 (2009).

As used herein, the terms **MTHF** or **methyleneTHF** shall both refer to 5,10-Methylene-5,6,7,8-tetrahydrofolate.

As used herein, the terms **racemic methyleneTHF, CoFactor^{®}** or **[6R,S]-5,10-methyleneTHF** shall all refer to the 1:1 diastereomeric mixture [6R,S]-5,10-Methylene-5,6,7,8-tetrahydrofolate.

As used herein, the terms arfolitixorin and [6R]-5,10-MTHF shall both refer to the single diastereomer, [6R]-5,10-methylenetetrahydrofolate.

As used herein, the terms IV or i.v. shall both mean **intravenous.**

As used herein, the term **ORR** shall refer to the Objective Response Rate, i.e. the proportion of patients with a reduction in tumor burden of a predefined amount. This shall be calculated as follows: ORR = Sum of partial responses plus complete responses as per RECIST 1.1 (a set of published rules (link: https://recist.eortc.org/recist-1-1-2/) that define when tumors in cancer patients progress during treatments, the responses being defined as:
**Complete Response (CR):**
   • Disappearance of all target lesions. Any pathological lymph nodes (whether target or non-target) must have reduction in short axis to <10 mm.
**Partial Response (PR):**
   • At least a 30% decrease in the sum of diameters of target lesions, taking as reference the baseline sum diameters.
**Progressive Disease (PD):**
   - At least a 20% increase in the sum of diameters of target lesions, taking as reference the smallest sum on study (this includes the baseline sum if that is the smallest on study).
   - In addition to the relative increase of 20%, the sum must also demonstrate an absolute increase of at least 5 mm. (Note: the appearance of one or more new lesions is also considered progression).
**Stable Disease (SD):**
• Neither sufficient shrinkage to qualify for PR nor sufficient increase to qualify for PD, taking as reference the smallest sum diameters while on study.

As used herein, the term **BSA** refers to Body Surface Area

As used herein, the term **CRC** refers to colorectal cancer. The term **mCRC** shall refer to metastatic colorectal cancer.

**FOLFOXIRI** and **FOLFIRINOX** are chemotherapy regimens consisting of leucovorin, fluorouracil, irinotecan, and oxaliplatin.

**FOLFIRI** is a chemotherapy regimen consisting of leucovorin, fluorouracil, and irinotecan.

**FOLFOX** is a chemotherapy regimen consisting of leucovorin, fluorouracil, and oxaliplatin.

**B6 vitamins** are a group of compounds that encompasses six interconvertible compounds (i.e., B6 vitamers), namely pyridoxine (PN); pyridoxamine (PM); pyridoxal (PL); and their respective 5'-phosphorylated forms, pyridoxine 5'-phosphate (PNP), pyridoxamine 5'-phosphate (PMP), and the cofactor pyridoxal 5'-phosphate (PLP).

### SUMMARY OF INVENTION

Arfolitixorin (Modufolin^{®}) is a new drug developed to increase the efficacy of the cytotoxic agent 5-fluorouracil (5-FU) and as a rescue drug after high-dose methotrexate treatment. Arfolitixorin (Modufolin^{®}), [6R]-5,10-methylenetetrahydrofolate, abbreviated herein as [6R]-5,10-MTHF, needs to be metabolically formed when using the widely used folate-based drugs leucovorin and levoleucovorin. Arfolitixorin (Modufolin^{®}), however, does not require metabolic activation to exert its effect and may therefore be suitable for all patients.

Applicant's previous work relating to "doublet" chemotherapeutic regimens (arfolitixorin plus 5-FU + either oxaliplatin or iriniotecan) has been described in i.a. WO 2019/037898 and WO 2019/037899.

Applicant has also investigated the more intense "triplet" chemotherapeutic regimens with a view to develop a more efficient and better tolerated folate-based regimen than the current state-of-the art FOLFOXIRI/FOLFIRINOX regimens (which employ leucovorin as the folate) comprising arfolitixorin, 5-FU, irinotecan, oxaliplatin + a monoclonal antibody (MAB).

According to the present invention, it has surprisingly been found that patients diagnosed with adenocarcinoma and further determined by genotype testing to be either *BRAF* mutation-positive or *KRAS* mutation-positive or both *BRAF* mutation-positive and *KRAS* mutation-positive, *i.e.* harboring *BRAF* mutation- and/or *KRAS* mutation-positive cancer tumors, may be treated according to a new B6 vitamin-enhanced triplet chemotherapeutic protocol over at least 16 weeks involving administration of arfolitixorin:
- A MAB selected from cetuximab, panitumumab or bevacizumab
- 5-fluorouracil, irinotecan, and oxaliplatin
- arfolitixorin ([6R]-5,10-MTHF)
- A B6 vitamin derivative, preferably selected from pyridoxamine (PM) or pyridoxine (PN)
by which treatment best ORRs (objective response rates) of >60%, such as >70% can be achieved, and which treatment induces fewer side effects and is thus better tolerated than standard-of-care FOLFOXIRI/FOLFIRINOX regimens.

Patients diagnosed with RAS-wildtype adenocarcinoma may also be successfully treated with the above protocol in which case the preferred MAB is cetuximab or panitumumab, while for patients diagnosed with RAS-mutated or BRAF-mutated adenocarcinoma, the preferred MAB is bevacizumab.

Accordingly, in a **first aspect** of the invention, arfolitixorin is provided for use in a human patient in the treatment of RAS-wildtype adenocarcinoma, which treatment comprises the following steps:
**On Day 1**
   a) administering a continuous IV infusion, either over 100 min ± 5 min containing 500 mg/m² of a monoclonal antibody selected from cetuximab and panitumumab (5 mg/m²/min), followed by
   b) administering a continuous IV infusion over 120 min ± 5 min containing 80 mg/m² oxaliplatin, followed by
   c) administering a continuous IV infusion over 30 min ± 3 min containing 180 ±5 mg/m² irinotecan, followed by
   d) administering a continuous IV infusion over 30 min ± 3 min containing 120 - 400 mg/m² arfolitixorin, followed by
   e) administering an IV infusion over 30 min ± 3 min containing 3000 - 6000 mg pyridoxine, followed by
   f) administering a continuous IV infusion over 120 min ± 10 min containing 625 ±5 mg/m² 5-fluorouracil (5-FU), followed by
   g) administering a continuous IV infusion containing 375 ±5 mg/m² 5-fluorouracil over 22 ± 1 hour, followed by
**On Day 2**
   h) administering a continuous IV infusion over 30 min ± 3 min containing 120 - 400 mg/m² arfolitixorin, followed by
   i) administering an IV infusion over 30 min ± 3 min containing 3000 - 6000 mg pyridoxine, followed by
   j) administering a continuous IV infusion over 120 min ± 10 min containing 625 ±5 mg/m² 5-FU, followed by
   k) administering a continuous IV infusion containing 375 mg/m² 5-FU over 22 ± 1 hour,
   wherein said human patient has been found by genotype testing to have RAS-wildtype adenocarcinoma, and wherein all steps a) - k) are repeated every 2 weeks for a total treatment period of at least 16 weeks or until termination of the treatment.

Further, in a **second aspect** of the invention, arfolitixorin is provided for use in a human patient in the treatment of RAS-mutated and/or *BRAF* mutated adenocarcinoma, which treatment comprises the following steps:
**On Day 1**
   a) administering a continuous IV infusion over 90 min ± 5 min containing 5 mg/kg bevacizumab, followed by
   b) administering a continuous IV infusion over 120 min ± 5 min containing 80 mg/m² oxaliplatin, followed by
   c) administering a continuous IV infusion over 30 min ± 3 min containing 180 ±5 mg/m² irinotecan, followed by
   d) administering a continuous IV infusion over 30 min ± 3 min containing 120 - 400 mg/m² arfolitixorin, followed by
   e) administering an IV infusion over 30 min ± 3 min containing 3000 - 6000 mg pyridoxine, followed by
   f) administering a continuous IV infusion over 120 min ± 10 min containing 625 ±5 mg/m² 5-fluorouracil (5-FU), followed by
   g) administering a continuous IV infusion containing 375 ±5 mg/m² 5-fluorouracil over 22 ± 1 hour, followed by
**On Day 2**
   h) administering a continuous IV infusion over 30 min ± 3 min containing 120 - 400 mg/m² arfolitixorin, followed by
   i) administering an IV infusion over 30 min ± 3 min containing 3000 - 6000 mg pyridoxine, followed by
   j) administering a continuous IV infusion over 120 min ± 10 min containing 625 ±5 mg/m² 5-FU, followed by
   k) administering a continuous IV infusion containing 375 mg/m² 5-FU over 22 ± 1 hour,
   wherein said human patient and has been found by genotype testing to have RAS-mutated and/or BRAF mutated adenocarcinoma, and wherein all steps a) - k) are repeated every 2 weeks for a total treatment period of at least 16 weeks or until termination of the treatment.

A treatment course comprises a two-day protocol as outlined above, which course is then repeated every 14 days. Assessment of antitumor efficacy is performed after each phase of 4 courses, i.e., before the 5^{th} course, before the 9^{th} course, etc.

The treatment based on either of the two protocols according to the first or second aspect of the present invention may in principle be terminated "for any reason", such as e.g. by a patient decision or a decision taken by the responsible medical person, i.a. due to disease progression or adverse events. Furthermore, the protocols may be interrupted by treatment holidays and the like. Finally the responsible medical person may decide on a fixed number of treatment cycles.

### DESCRIPTION OF FIGURES

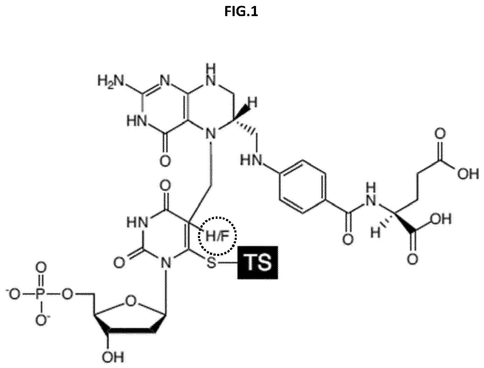

**Fig. 1****.** Ternary complex formed between [6R]-5,10-methylene-THF, thymidylate synthase, and 2'-deoxy-uridine-5'-monophosphate, 2-dUMP or 5-fluoro-2'-deoxy-uridine-5'-monophosphate, 5-FdUMP. The dotted circle marks the difference between 2-dUMP and 5-FdUMP. THF: tetrahydrofolate; TS: thymidylate synthase. (Adapted from Danenberg 2016)

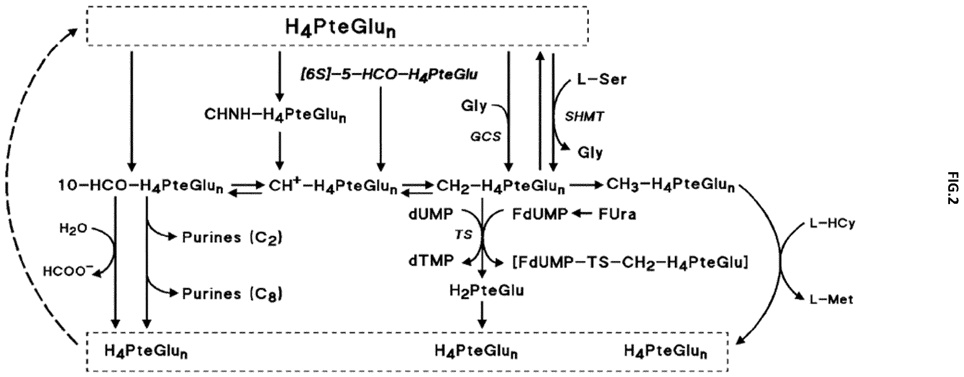

**Fig. 2****.** Selected pathways of folates and FdUMP-mediated thymidylate synthase (TS) inhibition. *Folates:* **H2PteGlu:** 7,8-dihydro pteroylglutamate; **H4PteGlu:** 5,6,7,8- tetrahydro pteroylglutamate; **CH2-H4PteGlu:** 5,10-methylene tetrahydro pteroylglutamate; **CH3-H4PteGlu:** 5-methyl tetrahydro pteroylglutamate; **CH⁺-H4PteGlu:** 5,10-methenyl tetrahydropteroylglutamate; **10-HCO-H4PteGlu:** 10-formyl tetrahydropteroylglutamate; **CHNH-H4PteGlu:** 5-formimino tetra hydro pteroylglutamate; **[6S]-5-HCO-H4PteGlu:** [6S]-5-formyl tetrahydro pteroylglutamate ([6S]-folinic acid). *Enzymes:* **TS,** thymidylate synthase; **SHMT,** serine hydroxymethyltransferase (pyridoxal 5'-phosphate-dependent enzyme, including the cytoplasmic SHMT1 and the mitochondrial SHMT2 isoforms); **GCS,** glycine cleavage system (mitochondrion). *Other compounds and substances involved in TS inhibition:* **dUMP,** deoxy uridine monophosphate; **dTMP,** thymidine monophosphate; **L-Ser,** L-serine; **Gly,** glycine; **L-**
**HCy,** L-homocysteine; **L-Met,** L-methionine; **HCOO⁻,** formate; **FUra,** 5-fluorouracil (5-FU); **FdUMP,** fluorodeoxyuridine monophosphate; **[FdUMP-TS-CH2-H4PteGlu],** the ternary complex resulting in inhibition of TS (see also fig.1). Synthesis of methylene tetrahydrofolate from THF results mainly from the transfer of Cβ of serine to THF catalyzed by SHMT, which is a ubiquitous pyridoxal 5'-phosphate (PLP)-dependent enzyme that is the major source of one-carbon units for cellular metabolism. (Figure Adapted from Machover 2022)

### DETAILED DESCRIPTION OF THE INVENTION

Arfolitixorin has been in development for a number of years and has been studied in several clinical studies. During one of these studies (the Phase I/IIa study ISO-CC-005) it was surprisingly discovered in December 2017 that administration of [6R]-MTHF (arfolitixorin) and 5-FU according to a particular treatment regimen over a treatment period of at least 8 weeks lead to a prevention or retarding of the progression in a human of solid tumors. No statistically significant progression of said solid tumors was observed between 8 and 16 weeks after initiating treatment. These results are discussed i.a. in applicant's international patent application WO 2019/037898 and WO 2019/037899.

The completion of the study was announced in January 2020. In total, 105 patients were included in the study. The data showed a best overall response rate (ORR) of 55 %. These patients had been treated with the "doublet" regimens comprising 120 mg/m² arfolitixorin and 5-fluorouracil (5-FU) with either irinotecan or oxaliplatin (ARFIRI/ARFOX). Out of the 31 patients, 17 were treated with an ARFOX regimen.

In addition to these doublet folate-based chemotherapeutic regimens (ARFIRI/ARFOX), applicant has been interested in developing improved versions of the more intensive "triplet regimens", such as the classical FOLFOXIRI/FOLFIRINOX (which employ leucovorin as the folate) i.e. versions comprising arfolitixorin, 5-FU, irinotecan and oxaliplatin, since on the one hand many patients with metastatic adenocarcinoma would benefit from such a high-intensity treatment whilst on the other hand a large proportion of such patients would not be eligible to high-intensity treatment due to the accompanying side-effects associated with the classical triplet strategies.

Leucovorin and I-leucovorin, both currently approved for treatment of gastrointestinal and other cancers, need to be metabolically activated to [6R]-MTHF to potentiate the effect of 5-FU. This dependency on cellular activation provides a hurdle for reaching sufficient levels of the active [6R]-MTHF. Arfolitixorin treatment has been shown to result in [6R]-MTHF levels that are at least 3-4 times higher than with comparable treatment with leucovorin, and consequently generates the preconditions for a substantial increase in the efficacy of 5-FU. Secondly, the enzymatic activation is genetically regulated^{16,18}, and is therefore associated with variability between individuals and conditions. Arfolitoxirin is believed to be efficacious in a larger proportion of patients with less inter- and intra-individual variability. These differences may potentially turn out to be of significant importance for increased clinical efficacy in 5-FU based cancer treatment.

Applicant thus speculated that by augmenting the effect of the folate component in the classical triplet regimen by replacement of leucovorin with arfolitixorin, it might be possible to develop a better tolerated, and potentially also more efficient triplet chemotherapeutic regimen for the treatment of aggressive cancer forms, in particular treatment of adenocarcinoma with *BRAF* or *KRAS* mutations.

In the treatment of cancer with 5-fluorouracil, the administration of folates mechanistically leads to the formation of [6R]-5,10-methylene-tetrahydrofolate, and the increased concentration of this molecule leads to stabilization of the "ternary complex" comprising thymidylate synthase (TS), 2'-deoxy-uridine-5'-monophosphate (dUMP), and [6R]-5,10-methylene-tetrahydrofolate (Danenberg 2016).

The difference in a clinical setting in human patients between leucovorin and arfolitixorin has been analyzed by Taflin et al. in the Modelle 001 Phase 2 clinical study (TS-inhibition in Colorectal Liver Metastases Comparing Arfolitixorin and Calciumfolinate), the primary objective of which is to compare the TS-inhibtion capacity of the combination Arfolitixorin/5-FU in contrast to Calciumfolinate/5-FU in tumour, adjacent liver parenchyma and plasma in patients with liver metastases from colorectal cancer (Study Details | Modelle 001, TS-inhibition in Colorectal Liver Metastases Comparing Arfolitixorin and Calciumfolinate | ClinicalTrials.gov) (Taflin 2023).

[6R]-5,10-methylene-THF is the key endogenous one-carbon donor and a co-substrate of the TS enzyme for methylation of dUMP to 2'-deoxy-thymidine-5'-monophosphate (dTMP). This product is one of the necessary nucleotide substrates for DNA synthesis and repair, and is thus essential for cell division and life. 5-FU is known to exert its cytotoxic activity, to a major extent, through inhibition of the TS enzyme. Importantly, for either synthesis of dTMP, via TS catalysis, or inhibition of TS, by 5-FU, a ternary complex is formed between TS, [6R]-5,10-methylene-THF, and the respective third component, either dUMP or 5-fluoro-2'-deoxy-uridine-5'-monophosphate (5-FdUMP; see **Fig. 1****).**

The results from the Modelle-1 study showed that both 30 and 120 mg/m2 arfolitixorin resulted in higher concentrations of [6R]-5,10-methylene-THF (CH₂THF) in metastases, both before (p = 0.037 and p = 0.0003, respectively) and after (p = 0.031 and p = 0.0023, respectively) 5-FU treatment, as compared to 60 mg/m2 Leucovorin. Administration of 30 or 120 mg/m2 arfolitixorin resulted in a higher ratio of poly/monoglutamated folates compared to 60 mg/m2 Leucovorin after 5-FU administration (p = 0.045 and p = 0.0049, respectively). A dose-dependent TS inhibition was seen after administration of 30 mg/m2 arfolitixorin (54.2% ± 189) vs 120 mg/m2 arfolitixorin (83.8% ± 431) and both 30 and 120 mg/m2 arfolitixorin seemed to give a better mean TS inhibition than 60 mg/m2 Leucovorin (25.4% ± 301, p = 0.081 and p = 0.0026, respectively). Thus, significantly higher concentrations of [6R]-5,10-methylene-THF, ratio of poly-/monoglutamated CH₂THF and TS inhibition was found in colorectal liver metastases in patients receiving treatment with Arfo compared to standard Leucovorin therapy.

Folates belong to the group of Vitamin B micronutrients (Franco 2022) which are essential regulators of the so-called one-carbon metabolism. The one-carbon metabolism is a collection of cyclical metabolic pathways that orchestrates several metabolic processes which are required for the maintenance of intracellular DNA synthesis and methylation. The one carbon pathway is comprised of the folate cycle and the methionine cycle (see Fig.2) in which in particular folate, vitamin B12 and B6 play a role. Folic acid (pteroyl-L-glutamic acid) is an oxidized folate also known as vitamin B9 which is not active in humans, but is converted in the liver to the biologically active 7,8-dihydrofolate (DHF). Folic acid does not by itself increase the concentration of [6R]-5,10-methylene-THF enough to enhance FdUMP-mediated inhibition of TS, and is thus unable to modulate 5-FU cytotoxicity at physiological concentrations.

Experiments conducted in the human colon carcinoma HT29 and HCT116 cell lines, and in the murine leukemia L1210 cell line *in vitro* to investigate for interactions between 5-FU, leucovorin and the cofactor pyridoxal 5'-phosphate (PLP) on cell growth has shown that supplementation of cancer cells exposed to 5-FU with high concentrations of PLP and leucovorin strongly potentiated the cytotoxic activity of 5-FU in the three cell lines (Machover 2018).

In a later study (Machover 2021) five patients (2M, 3F) with advanced metastatic colorectal adenocarcinoma were treated according to the classical FOLFIRINOX triplet regimen (leucovorin, 5-FU, irinotecan, and oxaliplatin) supplemented with pyridoxine in high doses accompanying each administration of leucovorin plus 5-FU. Two patients had adenocarcinoma with wildtype (wt) RAS, three with *KRAS* mutations. One patient had initial resection of the primary tumor and four did not. One patient had relapse and underwent treatment again. The five patients had an average ECOG score of >3.

The two RAS wt cases received pyridoxin + cetuximab in addition to the standard FOLFIRINOX protocol, whereas the three cases with *KRAS* mutations only received pyridoxin in addition to the standard treatment.

Antitumor activity was found to be high for several patients, but due to the small scale of the study, lack of controls and varied disease history it is not clear whether the co-administration of pyridoxine has led to a significantly different result from what would have been expected from employing the standard FOLFIRINOX protocol ±cetuximab on the same group of patients. Assessment of toxicity associated with the pyridoxine-modified protocols was however of the same magnitude as expected for the standard protocols.

According to the present invention, it has surprisingly been found that patients diagnosed with adenocarcinoma and further determined by genotype testing to be either *BRAF* mutation-positive or *KRAS* mutation-positive or both *BRAF* mutation-positive and *KRAS* mutation-positive, *i.e.* harboring *BRAF* mutation- and/or *KRAS* mutation-positive cancer tumors, may be treated according to a new B6 vitamin-enhanced triplet chemotherapeutic protocol over at least 16 weeks involving administration of arfolitixorin:
- A MAB selected from cetuximab or bevacizumab
- 5-fluorouracil, irinotecan, and oxaliplatin
- arfolitixorin ([6R]-5,10-MTHF)
- A B6 vitamin selected from pyridoxamine (PM) or pyridoxine (PN)
by which treatment best ORRs (objective response rates) of >60%, such as >70% can be achieved, and which treatment induces fewer side effects and is thus better tolerated than standard-of-care FOLFOXIRI/FOLFIRINOX regimens.

Patients diagnosed with RAS-wildtype adenocarcinoma may also be successfully treated with the above protocol in which case the preferred MAB is cetuximab or panitumumab, while for patients diagnosed with RAS-mutated or BRAF-mutated adenocarcinoma, the preferred MAB is bevacizumab.

Accordingly, in **a first aspect** of the invention, arfolitixorin is provided for use in a human patient in the treatment of RAS-wildtype adenocarcinoma, which treatment comprises the following steps:
**On Day 1**
   a) administering a continuous IV infusion, either over 100 min ± 5 min containing 500 mg/m² of a monoclonal antibody selected from cetuximab and panitumumab (5 mg/m²/min), followed by
   b) administering a continuous IV infusion over 120 min ± 5 min containing 80 mg/m² oxaliplatin, followed by
   c) administering a continuous IV infusion over 30 min ± 3 min containing 180 ±5 mg/m² irinotecan, followed by
   d) administering a continuous IV infusion over 30 min ± 3 min containing 120 - 400 mg/m² arfolitixorin, followed by
   e) administering an IV infusion over 30 min ± 3 min containing 3000 - 6000 mg pyridoxine, followed by
   f) administering a continuous IV infusion over 120 min ± 10 min containing 625 ±5 mg/m² 5-fluorouracil (5-FU), followed by
   g) administering a continuous IV infusion containing 375 ±5 mg/m² 5-fluorouracil over 22 ± 1 hour, followed by
**On Day 2**
   h) administering a continuous IV infusion over 30 min ± 3 min containing 120 - 400 mg/m² arfolitixorin, followed by
   i) administering an IV infusion over 30 min ± 3 min containing 3000 - 6000 mg pyridoxine, followed by
   j) administering a continuous IV infusion over 120 min ± 10 min containing 625 ±5 mg/m² 5-FU, followed by
   k) administering a continuous IV infusion containing 375 mg/m² 5-FU over 22 ± 1 hour, wherein said human patient has been found by genotype testing to have RAS-wildtype adenocarcinoma, and wherein all steps a) - k) are repeated every 2 weeks for a total treatment period of at least 16 weeks or until termination of the treatment.

In an embodiment of the first aspect of the present invention, the human patient has colorectal adenocarcinoma.

In another embodiment of the first aspect, the human patient is previously untreated.

In another embodiment of the first aspect, the human patient receives cetuximab on Day 1. In another embodiment, the human patient receives panitumumab on Day 1.

In yet another embodiment, the total treatment period is at least 20 weeks, such as at least 24 weeks.

Further, in **a second aspect** of the invention, arfolitixorin is provided for use in a human patient in the treatment of RAS-mutated and/or BRAF mutated adenocarcinoma, which treatment comprises the following steps:
**On Day 1**
   a) administering a continuous IV infusion over 90 min ± 5 min containing 5 mg/kg bevacizumab, followed by
   b) administering a continuous IV infusion over 120 min ± 5 min containing 80 mg/m² oxaliplatin, followed by
   c) administering a continuous IV infusion over 30 min ± 3 min containing 180 ±5 mg/m² irinotecan, followed by
   d) administering a continuous IV infusion over 30 min ± 3 min containing 120 - 400 mg/m² arfolitixorin, followed by
   e) administering an IV infusion over 30 min ± 3 min containing 3000 - 6000 mg pyridoxine, followed by
   f) administering a continuous IV infusion over 120 min ± 10 min containing 625 ±5 mg/m² 5-fluorouracil (5-FU), followed by
   g) administering a continuous IV infusion containing 375 ±5 mg/m² 5-fluorouracil over 22 ± 1 hour, followed by
**On Day 2**
   h) administering a continuous IV infusion over 30 min ± 3 min containing 120 - 400 mg/m² arfolitixorin, followed by
   i) administering an IV infusion over 30 min ± 3 min containing 3000 - 6000 mg pyridoxine, followed by
   j) administering a continuous IV infusion over 120 min ± 10 min containing 625 ±5 mg/m² 5-FU, followed by
   k) administering a continuous IV infusion containing 375 mg/m² 5-FU over 22 ± 1 hour, wherein said human patient and has been found by genotype testing to have RAS-mutated and/or BRAF mutated adenocarcinoma, and wherein all steps a) - k) are repeated every 2 weeks for a total treatment period of at least 16 weeks or until termination of the treatment.

A treatment course comprises a two-day protocol as outlined above, which course is then repeated every 14 days. Assessment of antitumor efficacy is performed after each phase of 4 courses, i.e., before the 5^{th} course, before the 9^{th} course, etc.

The treatment based on either of the two protocols according to the first or second aspect of the present invention may in principle be terminated "for any reason", such as e.g. by a patient decision or a decision taken by the responsible medical person, i.a. due to disease progression or adverse events. Furthermore, the protocols may be interrupted by treatment holidays and the like. Finally the responsible medical person may decide on a fixed number of treatment cycles.

In an embodiment of the second aspect, the patient has *KRAS* mutated adenocarcinoma. In another embodiment, the patient has *BRAF* mutated adenocarcinoma. In a further embodiment of the second aspect, the human patient has colorectal adenocarcinoma.

In another embodiment of the second aspect, the human patient is previously untreated.

In yet another embodiment of the second aspect, the total treatment period is at least 20 weeks, such as at least 24 weeks.

Throughout the present application the treatment regimen according to the first aspect is referred to as the "ARFOXIRI + cet/pan" protocol, and the treatment regimen according to the second aspect is referred to as the "ARFOXIRI + bev" protocol.

The treatment based on the two above ARFOXIRI protocols may in principle be terminated "for any reason", such as e.g. by a patient decision or a decision taken by the responsible medical person, i.a. due to disease progression or adverse events. Furthermore, the ARFOXIRI protocols may be interrupted by treatment holidays and the like. Finally, the responsible medical person may decide on a fixed number of treatment cycles.

In CRC patients the *KRAS* mutation status is traditionally determined by tumor sample analysis. This requires biopsy or resection surgery, and the subsequent analysis (extraction of genomic DNA from the tumor biopsy and analysis for mutations using dPCR) often takes weeks to complete. This creates problems in clinical situations which require urgent treatment based on the mutation status of the patient.

However, several studies the past 5-10 years have demonstrated that genotype testing by analysis of circulating, cell-free tumor DNA (ctDNA) in plasma or serum samples is becoming increasingly accurate and thus important as a non-invasive and fast alternative or supplement to tumor sample analysis. The method is also referred to as "Liquid Biopsy" analysis. Cell-free DNA (cfDNA) is fragmented DNA that is found in the non-cellular blood components.

Among tumor patients, ctDNA is 150-200 base pair fragments that are released by tumor cells into the bloodstream and represents a small fraction of the total cfDNA. Importantly, ctDNA retains epigenetic characteristics and carries tumor-specific mutations that can be detected in peripheral blood (Bi 2020). Analysis of ctDNA in plasma is based on sequencing assays, see eg Finkle 2021.

Similarly, another study (Bachet 2018) involving 329 patients with detectable ctDNA (at least one mutation or one methylated biomarker) showed an accuracy of 94.8% (95% CI, 91.9% to 97.0%) between the RAS mutation status in plasma and tissue. The absence of liver metastases also here was the main clinical factor associated with inconclusive ctDNA results. Analysis of ctDNA ("liquid biopsy" analysis ) is thus deemed an important tool for determining the relevant patient group according to the first or second aspect of the present invention.

Accordingly, in embodiments of the invention, arfolitixorin is provided for use in a human patient in the treatment of colorectal adenocarcinoma, which treatment comprises steps a) - k) according to the first or second aspect of the invention, wherein the human patient has been found either by traditional tumor tissue analysis or by ctDNA analysis to have either RAS mutation-positive or *RAS* wildtype (wt) metastatic colorectal cancer.

In preferred embodiments of any of the aspects of the invention, arfolitixorin (6R-MTHF) is employed as a solid form which is soluble in water, such as a lyophilizate or a salt, optionally stabilized by one or more suitable excipients and/or antioxidants such as citric acid or ascorbic acid or salt forms thereof.

In other preferred embodiments of any of the aspects of the invention the lyophilizate of arfolitixorin is reconstituted in aqueous media.

In other preferred embodiments of any of the aspects of the invention the lyophilizate of arfolitixorin is prepared from 6R-MTHF hemisulfate salt.

In other preferred embodiments of any of the aspects of the invention the lyophilizate is prepared from 6R-MTHF hemisulfate salt and trisodium citrate dihydrate.

### EXAMPLES

### Example 1

Arfolitixorin (former Modufolin^{®}) is a folate-based biomodulator developed by applicant to improve the outcome of a range of antimetabolite treatments used within oncology. One of the therapeutic areas of specific interest included in the development program of arfolitixorin is as biomodulator of 5-fluorouracil (5-FU) activity in standard treatment regime for advanced, metastatic CRC, such as Stage IV. A stable lyophilizate formulation of the naturally occurring diastereoisomeric folate [6R]-5,10-MTHF has successfully been made from the drug substance arfolitixorin hemisulfate. The drug substance arfolitixorin hemisulfate in itself was found to be an unexpectedly stable salt of the otherwise very unstable naturally occurring [6R]-5,10-MTHF. As mentioned in the background section of the present application, [6R]-5,10-methylenetetrahydrofolate, abbreviated herein as [6R]-5,10-MTHF, needs to be metabolically formed when using the widely used folate-based drugs leucovorin and levoleucovorin. Arfolitixorin, however, does not require metabolic activation to exert its effect and may be directly involved in the formation of the FdUMP TS ternary complex discussed hereinabove.

The below **Table 1** shows the treatment protocol for the employed chemotherapy agents (cetuximab/panitumumab, oxaliplatin, irinotecan, 5-fluorouracil, pyridoxine and arfolitixorin) employed for patients with RAS-wildtype colorectal adenocarcinoma:

**Table 1**

| | **Drug** | **Planned dose** | | | **Mode** |
|---|---|---|---|---|---|
| | | **1st level** | **2nd level** | **3rd level** | |
| | **cetuximab or panitumumab / ^{a}** | **500 mg/m²** | **n/a** | **n/a** | **iv 100 min** |
| **Day 1** | **oxaliplatin ^{b}** | **80 mg/m²** | **n/a** | **n/a** | **iv 120 min** |
| | **irinotecan ^{b}** | **180 mg/m²** | **n/a** | **n/a** | **iv 30 min** |
| | **[6R]-MTHF (No1) ^{d}** | **120-200 mg/m²** | **300 mg/m²** | **400 mg/m²** | **iv 30 min** |
| | **pyridoxine (No1) ^{e}** | **3000 mg** | **4500 mg** | **6000 mg** | **iv 30 min** |
| | **5-FU (No1)^{c}** | **625 mg/m²** | **n/a** | **n/a** | **iv 120 min** |
| | **5-FU (No2) ^{c}** | **375 mg/m²** | **n/a** | **n/a** | **iv 22 hrs** |

| | **Drug** | **Planned dose** | | | **Mode** |
|---|---|---|---|---|---|
| | | **1st level** | **2nd level** | **3rd level** | |
| **Day 2** | **[6R]-MTHF (No 2) ^{d}** | **120-200 mg/m²** | **300 mg/m²** | **400 mg/m²** | **iv 30 min** |
| | **pyridoxine (No 2) ^{e}** | **3000 mg** | **4500 mg** | **6000 mg** | **iv 30 min** |
| | **5-FU (No3) ^{c}** | **625 mg/m²** | **n/a** | **n/a** | **iv 120 min** |
| | **5-FU (No4) ^{c}** | **375 mg/m²** | **n/a** | **n/a** | **iv 22 hrs** |

| | | | | | |
|---|---|---|---|---|---|
| ^{a} Each two-day course of chemotherapy is preceded by cetuximab or panitumumab at 500 mg/m² iv over 100 min ^{b} Doses of oxaliplatin and irinotecan are adjusted according to tolerance. The dose of oxaliplatin is decreased by 20% in case of Grade 1 SPN, and the drug is suspended in case of ≥ Grade 2 SPN ^{c}According to tolerance in previous courses, the daily dose of 5-FU can be increased by 10% increments from one course to the next (*i.e*., C1: 80%; C2: 90%; C3: 100%; C4: 110%; C5:120% as maximum) ^{d,e}Increments from one dose level to next (e.g. 120-200 → 300 → 400 mg/m²) are performed in successive groups of 5 patients in absence of toxicity. | | | | | |

The below **Table 2** shows the treatment protocol for the employed chemotherapy agents (bevacizumab, oxaliplatin, irinotecan, 5-fluorouracil, pyridoxine and arfolitixorin) employed for patients with *RAS-* or BRAF-mutated colorectal adenocarcinoma:

**Table 2**

| | **Drug** | **Planned dose** | | | **Mode** |
|---|---|---|---|---|---|
| | | **1s level** | **2nd level** | **3rd level** | |
| **Day 1** | **bevacizumab ^{a}** | **5 mg/kg** | **-** | **-** | **iv 90 min** |
| | **oxaliplatin ^{b}** | **80 mg/m²** | **n/a** | **n/a** | **iv 120 min** |
| | **irinotecan ^{b}** | **180 mg/m²** | **n/a** | **n/a** | **iv 30 min** |
| | **[6R]-MTHF (No1) ^{d}** | **120-200 mg/m²** | **300 mg/m²** | **400 mg/m²** | **iv 30 min** |
| | **pyridoxine (No1) ^{e}** | **3000 mg** | **4500 mg** | **6000 mg** | **iv 30 min** |
| | **5-FU (No1)^{c}** | **625 mg/m²** | **n/a** | **n/a** | **iv 120 min** |
| | **5-FU (No2) ^{c}** | **375 mg/m²** | **n/a** | **n/a** | **iv 22 hrs** |

| | **Drug** | **Planned dose** | | | **Mode** |
|---|---|---|---|---|---|
| | | **1st level** | **2nd level** | **3rd level** | |
| **Day 2** | **[6R]-MTHF (No 2) ^{d}** | **120-200 mg/m²** | **300 mg/m²** | **400 mg/m²** | **iv 30 min** |
| | **pyridoxine (No 2) ^{e}** | **3000 mg** | **4500 mg** | **6000 mg** | **iv 30 min** |
| | **5-FU (No3) ^{c}** | **625 mg/m²** | **n/a** | **n/a** | **iv 120 min** |
| | **5-FU (No4) ^{c}** | **375 mg/m²** | **n/a** | **n/a** | **iv 22 hrs** |

| | | | | | |
|---|---|---|---|---|---|
| ^{a} Each two-day course of chemotherapy is preceded by bevacizumab at 5 mg/kg iv over 90 min ^{b} Doses of oxaliplatin and irinotecan are adjusted according to tolerance. The dose of oxaliplatin is decreased by 20% in case of Grade 1 SPN, and the drug is suspended in case of ≥ Grade 2 SPN ^{c}According to tolerance in previous courses, the daily dose of 5-FU can be increased by 10% increments from one course to the next (*i.e*., C1: 80%; C2: 90%; C3: 100%; C4: 110%; C5:120% as maximum) ^{d , e} Increments from one dose level to next (e.g. 120-200 → 300 → 400 mg/m²) are performed in successive groups of 5 patients in absence of toxicity. | | | | | |

### Example 2

The effectiveness of arfolitixorin vs. leucovorin in Thymidylate Synthase inhibition and in achieving high levels of [6R] 5,10-methylenetetrahydrofolate in metastatic tissue was investigated in the Modelle-001 Phase II clinical trial named "A Single-blinded, Randomized Phase II study investigating the effects of two doses of Arfolitixorin compared to Calcium folinate together with 5-fluorouracil on thymidylate synthase in tumour and adjacent hepatic tissue for patients with liver metastases from colorectal cancer". Link: Study Details I Modelle 001, TS-inhibition in Colorectal Liver Metastases Comparing Arfolitixorin and Calciumfolinate I Clinical-Trials.gov.

**Background:** The chemotherapeutic drug 5-fluorouracil (5-FU) is one of the cornerstones in treatment of colorectal metastases, by targeting the vital enzyme thymidylate synthase (TS). The cytotoxicity of the treatment results from inhibition of TS activity by the 5-FU metabolite FdUMP which forms a stable ternary complex with TS. The effect of 5-FU can be enhanced by reduced folates, most often racemic leucovorin (LV), that strengthens the complex. Polyglutamed folates are better stabilizers of the ternary complex than monoglutamated forms. In contrast to LV, arfolitixorin ([6R] 5,10-methylenetetrahydrofolate) (Arfo) does not require enzymatic metabolic activation because it constitutes the active folate of the ternary complex. A hypothesis is therefore that patients who are uncapable of metabolizing LV could benefit from Arfo administration.

**Aim:** The Modelle-001 trial was designed to explore how a single intravenous bolus injection of Arfo as compared to LV in the form of calcium folinate, together with 5-FU affects the inhibition of TS and, further, to investigate how different forms and doses of folates affect folate concentration and polyglutamation in metastatic liver tissue.

**Patients and Methods:** Thirty adult patients with biopsy-verified colorectal cancer and liver metastases indicated for surgical removal were included at two Swedish University Hospitals. Both synchronous and metachronous liver metastases were accepted and most patients had received preoperative chemotherapy. The first six patients received a reduced 5-FU dosage (250 mg/m2), and an independent safety committee gave permission to escalate to full dosage. Twenty-four patients were randomized to receive 500 mg/m2 5-FU either in combination with 60 mg/m2 LV (LV60, n = 12), 30 mg/m2 Arfo (A30, n = 6) or 120 mg/m2 Arfo (A120, n = 6) as a bolus regimen (a slow i.v. injection for three minutes). Metastatic tissue was collected 60 minutes after folate administration, then 5-FU was given. After a 60-minute break, metastatic tissue was again collected. Folate levels and TS inhibition were determined by LC-MS/MS.

**Results:** The results showed that both A30 and A120 resulted in higher 5,10-methylenetetrahydrofolate (CH₂THF) concentrations in metastases, both before (p = 0.037 and p = 0.0003, respectively) and after (p = 0.031 and p = 0.0023, respectively) 5-FU treatment, as compared to LV60. Administration of A30 or A120 resulted in a higher ratio of poly/monoglutamated folates compared to LV60 after 5-FU administration (p = 0.045 and p = 0.0049, respectively). A dose-dependent TS inhibition was seen after administration of A30 (54.2% ± 189) vs A120 (83.8% ± 431) and both A30 and A120 seemed to give a better mean TS inhibition than LV60 (25.4% ± 301, p = 0.081 and p = 0.0026, respectively). The perioperative chemotherapy was well tolerated, and no Suspected Unexpected Serious Adverse Reaction occurred.

**Conclusions:** Significantly higher CH₂-THF concentration, ratio of poly-/monoglutamated MeTHF and TS inhibition was found in colorectal liver metastases in patients receiving bolus injections of Arfo compared to standard bolus LV therapy. Thus, the results of the Modelle-001 study suggest that treatment with 5-FU in combination with Arfo could lead to more effective clinical outcome which would be of great importance.

### TREATMENT AND MATERIALS

**Arfolitixorin** ([6R]-5,10-Methylene-tetrahydrofolic acid, [6R]-MTHF) is formulated as a lyophilized powder containing 100 mg per vial (calculated as free acid). Dosing: [6R]-MTHF may be administered as a bolus regimen (a slow i.v. injection for three minutes) of 30-120 mg/m2 or as an infusion regimen (injections at a fixed dose of 120-200, 300 or 400 mg/m² administered over 30 min after administration of 5-FU (see below) on both Day 1 and Day 2 in each treatment cycle in all cycles of the study. For the first cycle 200 mg/m² is given followed by 300 mg/m² and finally a dose of maximum 400 mg/m² is given for all subsequent cycles). The infusion regimen will be repeated every second week for at least eight (8) cycles, i.e. sixteen (16) weeks.

**5-FU (5-fluorouracil)** is formulated as injection solution. Dosing: 5-FU is administered as infusions on Day 1 and Day 2 in each treatment cycle after injection of pyridoxine (see below). 5-FU will be administered at a fixed dose of 625 mg/m² over 120 min after finalizing the injection of pyridoxine, and again administered as an infusion of 375 mg/m² over 22 hrs The treatment will be repeated every second week for at least eight (8) cycles, i.e. sixteen (16) weeks.

**Oxaliplatin** is formulated as a concentrated infusion solution. Dosing: Oxaliplatin will be administered as an i.v. infusion of 80 mg/m² over 120 minutes on Day 1 in each treatment cycle after cetuximab/bevacizumab administration (see below), and repeated every second week for at least eight (8) cycles, i.e. sixteen (16) weeks. For the first cycle, 80% of the dose (64 mg/m²) is given before increasing to 100% (80 mg/m²) for subsequent cycles. Caution will be taken regarding toxicity associated with administration that may affect rate of infusion (e.g. grade ≤ 2 allergy, laryngopharyngeal dysesthesias, and laryngeal spasm). In such cases, rate of oxaliplatin administration should be prolonged in following cycles according to clinical practice recommendations.

**Irinotecan** is formulated as a concentrated infusion solution. Dosing: Irinotecan will be administered as an i.v. infusion of 180 mg/m² over 30 minutes on Day 1 in each treatment cycle after oxaliplatin administration and repeated every second week for at least eight (8) cycles, i.e. sixteen (16) weeks. For the first cycle, 80% of the dose (144 mg/m²) is given before increasing to 100% (180 mg/m²) for subsequent cycles. Caution will be taken regarding early toxicity (within 24 hours) associated with irinotecan administration, i.e. acute cholinergic syndrome, characterized by early diarrhoea, emesis, diaphoresis, abdominal cramping, and, less commonly, hyperlacrimation and rhinorrhoea. In such cases, the use of anticholinergics according to clinical practice recommendations is necessary.

**Avastin** (bevacizumab) is formulated as a concentrated infusion solution.

Dosing: Bevacizumab is administered at time = zero on Day 1 as an i.v. infusion during 90 minutes in each treatment cycle of the **ARFOXIRI** + **bev** protocol and repeated every second week for uat least eight (8) cycles, i.e. sixteen (16) weeks.

**Erbitux** (cetuximab) is formulated as a sterile, clear, colorless liquid of pH 7.0 to 7.4, which may contain a small amount of easily visible, white, amorphous cetuximab particulates. ERBITUX is supplied at a concentration of 2 mg/mL in either 100 mg (50 mL) or 200 mg (100 mL), single-use vials. Cetuximab is formulated in a solution with no preservatives, which contains 8.48 mg/mL sodium chloride, 1.88 mg/mL sodium phosphate dibasic heptahydrate, 0.41 mg/mL sodium phosphate monobasic monohydrate, and Water for Injection, USP.

Dosing: cetuximab is administered at time = zero on Day 1 as an i.v. infusion of 180 mg/m² during 100 minutes in each treatment cycle of the **ARFOXIRI** + **cet/pan** protocol and repeated every second week for at least eight (8) cycles, i.e. sixteen (16) weeks.

**Vectibix** (panitumumab) is a sterile, colorless, pH 5.6 to 6.0 liquid for intravenous (IV) infusion, which may contain a small amount of visible translucent-to-white, amorphous, proteinaceous, panitumumab particulates. Each single-use 5 mL vial contains 100 mg of panitumumab, 29 mg sodium chloride, 34 mg sodium acetate, and Water for Injection, USP. Each single-use 10 mL vial contains 200 mg of panitumumab, 58 mg sodium chloride, 68 mg sodium acetate, and Water for Injection, USP. Each single-use 20 mL vial contains 400 mg of panitumumab, 117 mg sodium chloride, 136 mg sodium acetate, and Water for Injection, USP.

Dosing: panitumumab is administered at time = zero on Day 1 as an i.v. infusion of 180 mg/m² during 100 minutes in each treatment cycle of the **ARFOXIRI** + **cet/pan** protocol and repeated every second week for at least eight (8) cycles, i.e. sixteen (16) weeks.

**Pyridoxine** hydrochloride is a colorless or white crystal or a white crystalline powder. One gram dissolves in 5 mL of water. It is stable in air and is slowly affected by sunlight. Pyridoxine Hydrochloride Injection, USP is formulated in 5 ml vials of 250 mg pyridoxine hydrochloride in water (50 mg/ml).

Dosing: pyridoxine is administered right after arfolitixorin administration as an i.v. infusion of 3000 - 6000 mg/m² during 30 minutes on both Day 1 and Day 2 in each treatment cycle and repeated every second week for at least eight (8) cycles, i.e. sixteen (16) weeks. For the first cycle 3000 mg/m² is given followed by 4500 mg/m² and finally a dose of maximum 6000 mg/m² is given for all subsequent cycles.

Assessment of antitumor efficacy for each of the two above dosage protocols is performed after each phase of 4 courses (i.e., before the 5th course, before the 9th course, etc.). The following variables are assessed: Antitumor response rate (according to RECIST and PERCIST); Magnitude of response; Rapidity of response (tumor shrinkage); Pathologic responses when indicated; PFS/DFS; and assessment of toxicity.

### REFERENCES

1. F Caputo et al. BRAF-Mutated Colorectal Cancer: Clinical and Molecular Insights. Int. J. Mol. Sci. 2019, 20, 5369
2. F Bray, et al. Global cancer statistics 2018: GLOBOCAN estimates of incidence and mortality worldwide for 36 cancers in 185 countries. CA Cancer J. Clin. 2018, 68, 394-424.
3. American Cancer Society. Cancer Facts & Figures; American Cancer Society: Atlanta, GA, USA, 2016.
4. C Cremolini et al. Individual Patient Data Meta-Analysis of FOLFOXIRI Plus Bevacizumab Versus Doublets Plus Bevacizumab as Initial Therapy of Unresectable Metastatic Colorectal Cancer. Journal of Clinical Oncology 38(28) August 2020
5. K Van der Jeught et al. Drug resistance and new therapies in colorectal cancer. World J Gastroenterol 2018 September 14; 24(34): 3834-3848
6. J D Finkle et al., Validation of a liquid biopsy assay with molecular and clinical profiling of circulating tumor DNA. npj Precis. One. 5, 63 (2021).
7. J B Bachet et al., RAS mutation analysis in circulating tumor DNA from patients with metastatic colorectal cancer: the AGEO RASANC prospective multicenter study. Ann Oncol. 2018 May 1;29(5):1211-1219. doi: 10.1093/annonc/mdy061
8. A.Tsuji et al. The randomized phase II study of FOLFOXIRI plus cetuximab versus FOLFOXIRI plus bevacizumab as the first-line treatment in metastatic colorectal cancer with RAS wild-type tumors: The DEEPER trial (JACCRO CC-13). Journal of Clinical Oncology 39, no. 15_suppl (May 20, 2021) 3501-3501. (DOI: 10.1200/JCO.2021.39.15_suppl.3501)
9. G Patelli et al. Strategies to tackle RAS-mutated metastatic colorectal cancer. ESMO Open Cancer Horizons Volume 6 - Issue 3 - 2021 https://doi.org/10.1016/j.esmoop.2021.100156
10. OECD/World Health Organization (2018), "Five-year net survival for breast, cervical and colorectal cancer", in Health at a Glance: Asia/Pacific 2018: Measuring Progress towards Universal Health Coverage, OECD Publishing, Paris. DOl: https://doi.org/10.1787/health glance ap-2018-43-en
11. Alkader M S et al. Impact of KRAS Mutation on Survival Outcome of Patients With Metastatic Colorectal Cancer in Jordan. Cureus 15(1): e33736 (January 13, 2023) https://doi:10.7759/cureus.33736
12. P V Danenberg et al. Folates as adjuvants to anticancer agents: Chemical rationale and mechanism of action. Critical Reviews in Oncology/Hematology 106 (2016) 118-131
13. J Dierkes et al. Folic acid and Vitamin B6 supplementation and plasma homocysteine concentrations in healthy young women. Int J Vitam Nutr Res 1998;68(2):98-103.
14. B Shane. Folate and vitamin B12 metabolism: Overview and interaction with riboflavin, vitamin B6, and polymorphisms. Food and Nutrition Bulletin, vol. 29, no. 2 (supplement) © 2008, The United Nations University.
15. Y Song et al. Effect of Combined Folic Acid, Vitamin B6, and Vitamin B12 on Colorectal Adenoma. J Natl Cancer Inst 2012;104:1562-1575
16. XH Zhang et al. Vitamin B6 and colorectal cancer: Current evidence and future directions. World J Gastroenterol 2013; 19(7): 1005-1010 [PMID: 23467420 DOI: 10.3748/wjg.v19.i7.1005 ]
17. D Machover et al. Pharmacologic modulation of 5-fluorouracil by folinic acid and pyridoxine for treatment of patients with advanced breast carcinoma. Scientific Reports | (2022) 12:9079
18. D Machover et al. Pharmacologic modulation of 5-fluorouracil by folinic acid and high-dose pyridoxine for treatment of patients with digestive tract carcinomas. Scientific Reports | (2021) 11:12668
19. D Machover et al. Enhancement of 5-fluorouracil cytotoxicity by pyridoxal 5'-phosphate and folinic acid in tandem. J. Pharmacol. Exp. Ther. 366, 238-243 (2018).
20. Y Wong et al. Long-Term Survival of De Novo Stage IV HER2 Positive Breast Cancers. The Oncologist 2019;24:313-318.
21. Taflin et al. Thymidylate synthase inhibition in colorectal liver metastases can be improved with Arfolitixorin compared to Leucovorin. Poster presented on 4 October 2023 at the Folate Receptor Society meeting during the "8th International Symposium on Folate Biology and Therapeutics" Edith Macy Center Briarcliff Manor, New York October 2023.

## Claims

1. Arfolitixorin for use in a human patient in the treatment of adenocarcinoma, which treatment comprises the following steps:
**On Day 1**
a) administering a continuous IV infusion, either
i. containing 500 mg/m² cetuximab or panitumumab over 100 min ± 5 min
or
ii. containing 5 mg/kg bevacizumab over 90 min ± 5 min,
followed by
b) administering a continuous IV infusion over 120 min ± 5 min containing 80 mg/m² oxaliplatin, followed by
c) administering a continuous IV infusion over 30 min ± 3 min containing 180 mg/m² irinotecan, followed by
d) administering a continuous IV infusion over 30 min ± 3 min containing 120 - 400 mg/m² arfolitixorin, followed by
e) administering an IV infusion over 30 min ± 3 min containing 3000 - 6000 mg of a B6 vitamin selected from pyridoxamine or pyridoxine, followed by
f) administering a continuous IV infusion over 120 min ± 10 min containing 625 mg/m² 5-fluorouracil (5-FU), followed by
g) administering a continuous IV infusion containing 375 mg/m² 5-fluorouracil over 22
hours ± 1 hour, followed by
**On Day 2**
h) administering a continuous IV infusion over 30 min ± 3 min containing 120 - 400 mg/m² arfolitixorin, followed by
i) administering an IV infusion over 30 min ± 3 min containing 3000 - 6000 mg of a B6 vitamin selected from pyridoxamine or pyridoxine, followed by
j) administering a continuous IV infusion over 120 min ± 10 min containing 625 mg/m² 5-fluorouracil (5-FU), followed by
k) administering a continuous IV infusion containing 375 mg/m² 5-fluorouracil over 22 hours ± 1 hour,
wherein said patient has been found by genotype testing to have either Ras-WT, Ras-mutated and/or *BRAF* mutated adenocarcinoma, and wherein all steps a) - k) are repeated every 2 weeks for a total treatment period of at least 16 weeks or until termination of the treatment.

2. Arfolitixorin for use in a human patient according to claim 1, wherein said patient has Ras-WT adenocarcinoma.

3. Arfolitixorin for use in a human patient according to claim 2, wherein on Day 1 a continuous IV infusion containing 500 mg/m² cetuximab is administered over 100 min ± 5 min.

4. Arfolitixorin for use in a human patient according to claim 2, wherein on Day 1 a continuous IV infusion containing 500 mg/m² panitumumab is administered over 100 min ± 5 min.

5. Arfolitixorin for use in a human patient according to claim 1, wherein said patient has Ras- and/or BRAF-mutated adenocarcinoma.

6. Arfolitixorin for use in a human patient according to claim 5, wherein genotype testing has shown that the patient is KRAS mutation-positive.

7. Arfolitixorin for use in a human patient according to any one of claim 5 or 6, wherein genotype testing has shown that the patient is BRAF-mutation-positive.

8. Arfolitixorin for use in a human patient according to any one of claim 5 - 7, wherein on Day 1 a continuous IV infusion containing 5 mg/kg bevacizumab is administered over 90 min ± 5 min.

9. Arfolitixorin for use in a human patient according to any one of claim 1 - 8, wherein said adenocarcinoma is colorectal adenocarcinoma.

10. Arfolitixorin for use in a human patient according to any one of claim 1 - 9, wherein genotype testing is performed by dPCR analysis of tumor tissue.

11. Arfolitixorin for use in a human patient according to any one of claim 1 - 10, wherein said patient is previously untreated for adenocarcinoma.

12. Arfolitixorin for use in a human patient according to any one of the preceding claims wherein arfolitixorin is employed as a solid form which is soluble in water, such as a lyophilizate or a salt, optionally stabilized by one or more suitable excipients and/or antioxidants such as citric acid or ascorbic acid or salt forms thereof.

13. Arfolitixorin for use in a human patient according to any one of the preceding claims wherein 5-fluorouracil (5-FU) is replaced by a fluorinated pyrimidine base such as capecitabine (Xeloda), i.e. N4-pentyloxycarbonyl-5'-deoxy-5-fluorocytidine, tegafur, 5-fluoro-pyrimidinone, UFT, doxifluridine, 2'-deoxy-5 fluorouridine, 5'-deoxy-5-fluorouridine, 1-(2'-oxopropyl)-5-FU, and alkyl-carbonyl-5-FU, BOF-A2, ftorafur(TS-1), and S-1.

14. Arfolitixorin for use in a human patient according to any one of the preceding claims wherein treatment is terminated by a patient decision or a decision taken by the responsible medical person, i.a. due to disease progression, adverse events or treatment holidays.
